# EUROPEAN PATENT SPECIFICATION

(11) **EP 0 746 227 B1**
(45) Date of publication and mention of the grant of the patent: **07.07.2004**
(21) Application number: 95906689.5
(22) Date of filing: 22.12.1994
(51) Int. Cl.: A61B 5/00, A61B 10/00, A61B 17/20, A61B 17/22, A61D 1/02, A61M 29/00, A61M 37/00, B65D 81/00

(54) **SAMPLE COLLECTION**
PROBEN-ENTNAHME
COLLECTE D'ECHANTILLONS

(30) Priority: 30.12.1993 US 175949; 30.12.1993 US 175791
(43) Date of publication of application: 11.12.1996
(73) Proprietor: BOSTON SCIENTIFIC CORPORATION, Natick, MA 01760-1537 (US)
(72) Inventor: SAHATJIAN, Ronald A., Lexington, MA 02173 (US)
(74) Representative: Brunner, Michael John
(86) International application number: PCT/US1994/014790
(87) International publication number: WO 1995/017849

(56) References cited:
- EP-A- 0 399 712
- EP-A- 0 436 898
- WO-A-80/01353
- AU-B- 516 114
- FR-A- 2 344 296
- SU-A- 1 069 826
- US-A- 4 623 329
- US-A- 4 781 699
- US-A- 5 186 172
- US-A- 5 389 074

## Description

The invention relates to collecting bodily samples from a patient.

Samples of tissue, bodily fluids, etc. are frequently taken from patients for analysis to help in diagnosing disease or monitoring the progress of treatment. For example, samples of cells from the lungs or gastrointestinal tract are taken with a cytology brush. The brush is rubbed against tissue to scrape cells from the surface and collect them in the bristles. Other sampling techniques sever tissue from the body. For example, biopsies are taken with a needle device that penetrates the tissue and then severs a sample with a sharp cutting cannula. A biopsy forceps device is another example. This device is a catheter with a jaw-type cutter at its end. The catheter is threaded through an endoscope to a position deep within the body where it bites a sample of tissue from a desired location. Samples of tissue are taken from within blood vessels using an artherectomy cutter. An artherectomy cutter is a catheter that can be threaded through a blood vessel to a desired site. A cutting member is provided at the end of the catheter. The cutting member can be pressed against a desired site in the blood vessel, such as the site of a vascular occlusion resulting from the build up of plaque, and then actuated to sever occluding matter from the wall of the blood vessel. Samples of bodily fluids are typically drawn from body conduits.

EP-A-0 436 898 (US-A-5 213 577) discloses a device in which an inner tube 1 is surrounded by a plastic tube 10, wherein the plastic tube 10 has zones 2, 3 which are more flexible than the remainder of the plastic tube 10 (see '577 patent, col 2, lines 25-29). The device has an outlet 6 and inlet 7 that may have the form of "short tubular members extending through the walls of tube 1" or "simple openings" in the wall of the tube.

SU-A-1069826 discusses an endotracheal tube designed specifically for introducing pharmaceutical agents into the trachea. The disclosure relates generally to medical technology, specifically to attachments that are used for the incubation of pneumatic tracks and electrophoresis of pharmaceutical agents into adjacent tissues.

According to the invention there is provided a sample collection device for collecting a sample from a desired location in a patient, the sample collection device comprising a sampling probe in the form of an elongate catheter having:
a proximal portion that remains outside the body and a distal portion that can be located at the desired location within the body;
an expandable member located at said distal portion, the expandable member being capable of being selectively expanded to larger diameters and contracted to smaller diameter;
a source of suction force located at said proximal portion of the device;
a membrane located on at least a part of an outside surface of said expandable member;
a sample storage space located in the interior of said membrane and communicating with source of suction force;
a plurality of support struts in the space to prevent the membrane from collapsing during application of the suction force; and
a plurality of openings extending through said membrane and communicating with the space in the interior of the membrane.

The device is used by positioning the expandable portion at a desired location within the blood vessel, taking the sample by exposing the membrane by expanding the expandable portion sufficient to place the membrane in proximity with the wall of the blood vessel so that the bodily sample is received by the membrane, contracting the expandable portion to smaller diameter so that the polymer does not contact the wall of the blood vessel, removing the catheter from the patient, and collecting the sample outside the body.

The space in the interior of the membrane is adapted to communicate with a suction device.

The membrane may be adapted such that suction forces draw the sample to be collected to the surface of the membrane.

The membrane may be adapted such that suction forces draw the sample to be collected through the openings into the space in the interior of the membrane.

In one embodiment the openings are adapted for the collection of cells and in another for the collection of tissue.

The device may include an analysis apparatus for facilitating analysis of said sample to determine physiological function.

The probe may be in the form of an elongate vascular catheter.

Alternatively, the probe may be in the form of an angioplasty catheter and the expandable member is in the form if an inflatable balloon, the membrane being disposed over said balloon.

The method of using the device may include removing the sample from the membrane by a flushing force back out through the openings, expanding the expandable portion to contact the membrane with the vessel wall, and moving the catheter axially while the membrane is in contact with the wall of the vessel to brush the membrane along the wall. The method may include positioning the expandable portion adjacent an occlusion in the vessel, simultaneously expanding the occlusion, and collecting the sample by expanding the expandable portion. The method can include performing angioplasty on an occluded region of the blood vessel, positioning the expandable portion adjacent the region at the region after the angioplasty, and collecting the sample from the region. The method also may include taking cells from the body and placing them back into the body.

The invention has many advantages. In embodiments, a sample of tissue or bodily fluid containing cells or chemical indicators of biological function can be collected from a site within a blood vessel in a low stress manner. This is an advantage since mechanically disturbing the vessel can cause further injury by inducing intimal proliferation (excessive scar forming) or dislodging portions of the occluding material on the vessel wall so that they enter the bloodstream.

Other features and advantages follow.

One example of a device according to the invention will now be described with reference to the accompanying drawings, in which:-

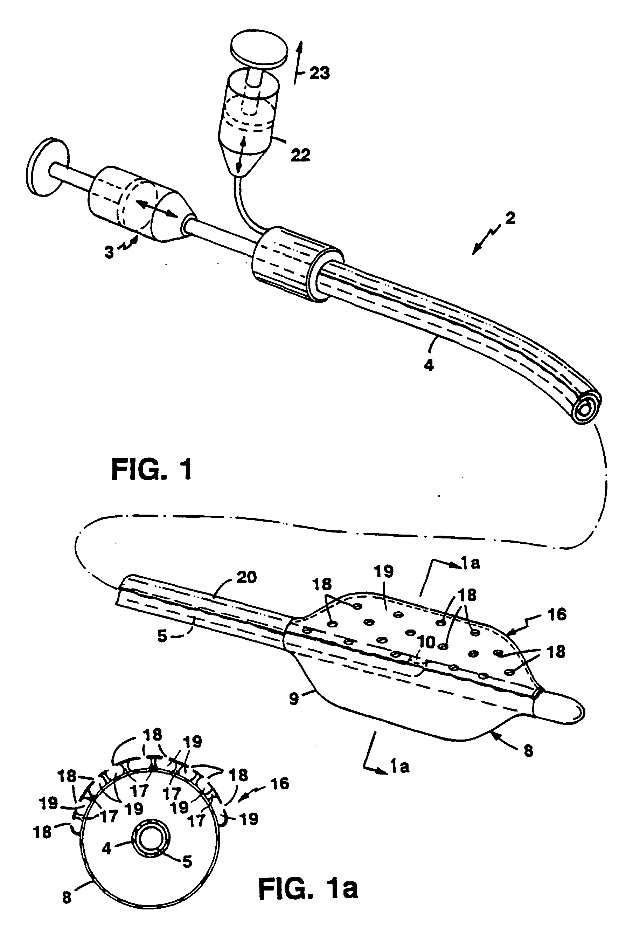

Figs. 1 to 6 illustrate the structure and use of an embodiment of the invention.

Referring to Fig. 1, a sample collection apparatus 2 includes a catheter 4 that is constructed to be threaded through a lumen of a blood vessel. The catheter 4 includes near its distal end an expandable portion, which may be, for example, a balloon 8. The balloon is inflated and deflated by injecting or withdrawing fluid from a source 3 through a lumen 5 in the catheter and a port 10 located within the balloon. The apparatus may include a protective sheath 30 that extends over the balloon while it is threaded into and out of the body. The balloon can be exposed from the sheath once the site is reached.

The sheath may be an introducer catheter of the type used to direct angioplasty catheters to the coronary arteries.

Referring as well to Fig. 1a, a cross-section along the line aa in Fig. 1, the apparatus also includes a membrane 16 on at least a portion of the outer surface of the balloon 8. The membrane includes a series of openings 18 that communicate with storage space 19 between the outer surface of the balloon and the inner surface of the membrane. The storage space 19 communicates with a lumen 20 that extends through the catheter to a source of suction force, e.g. a syringe 22, at the proximal end of the device. The membrane includes support struts 17 in the storage space to prevent the membrane from collapsing during suction.

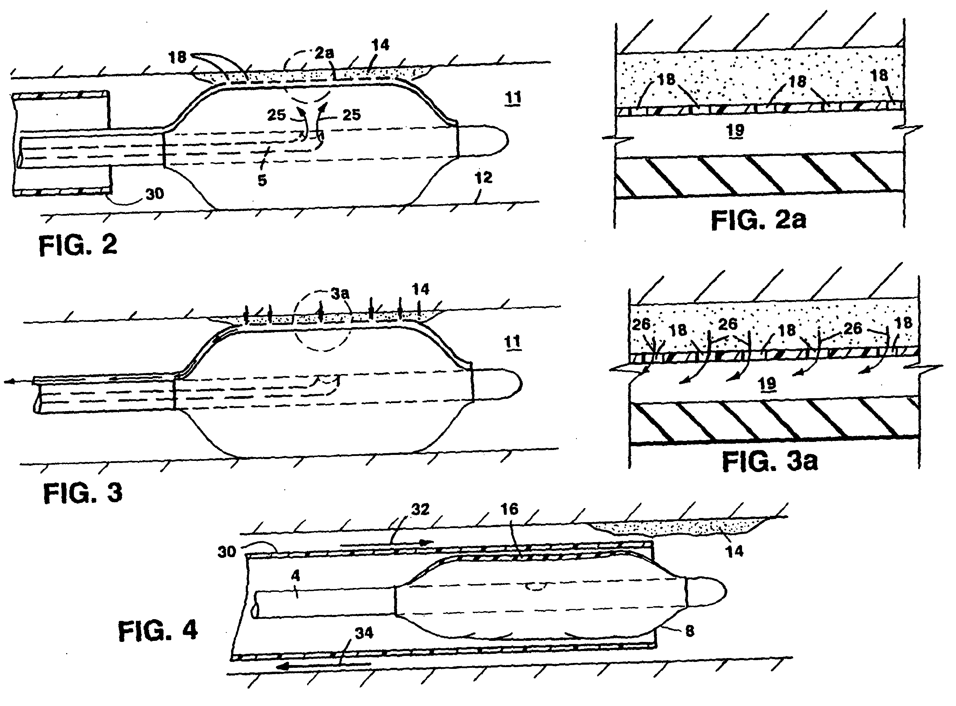

Referring to Fig. 2, an enlarged view of the distal end of the device, the balloon is positioned inside a blood 8 vessel 11 so the membrane 16 is adjacent a portion 14 of the vessel wall 12 that is diseased. For example, the portion 14 may be an occlusion caused by the build-up of plaque or the growth of smooth muscle cells, a condition known as intimal proliferation. The portion of the vessel may have already been treated by balloon catheter angioplasty so that the lumen is substantially open.

Referring as well to Fig. 2a, which is a greatly enlarged view of the area in the circle in Fig. 2, the balloon 8 is inflated by introducing inflation fluid (arrows 25) so that the outer surface of the membrane is in intimate contact with or a short distance from the surface of wall portion 14.

Referring to Figs. 3 and 3a, a suction force (created by actuating (arrow 23) syringe 22, see Fig. 1) draws bodily sample material from the wall portion 14 through the openings 18 into the storage space 19 (arrows 26).

Referring to Fig. 4, with the bodily sample material collected in the space 19, the balloon 8 is deflated to a small size. The protective sheath 30 may be slid axially over the balloon (arrow 32), and the catheter removed from the body (arrow 34). (The sheath may be a guiding catheter into which the sampling catheter is drawn.)

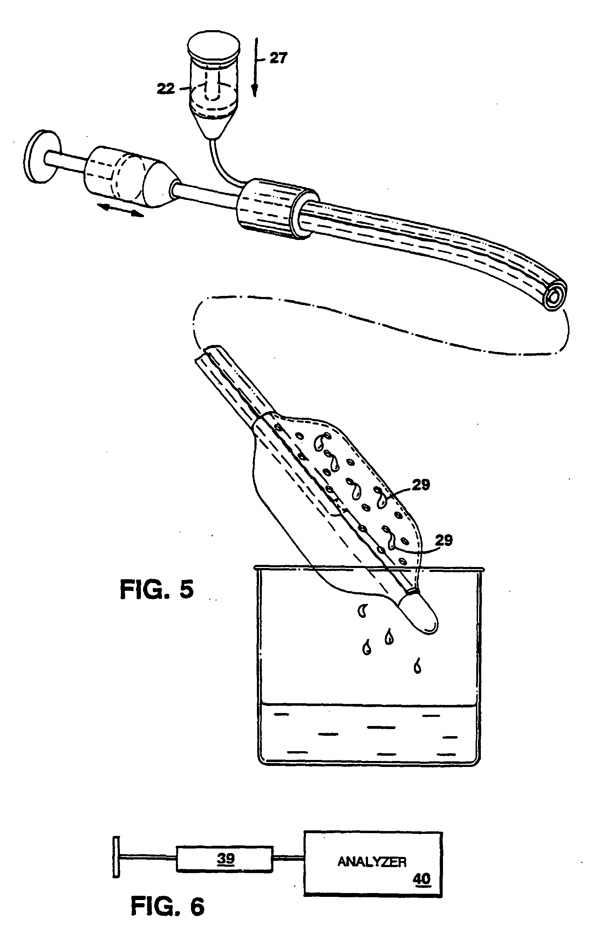

Referring to Fig. 5, outside the body, the bodily sample is removed from the space 19 by actuating the syringe 22 (arrow 27) to flush it from the space 19, back out of the openings 18 (arrow 29), and into a laboratory vessel 38. In other embodiments, the sample can be drawn to the body from the sample space, through the lumen 20, to be collected outside the body while the balloon is still in the body. Referring to Fig. 6, a sample of the bodily material is then placed, e.g., injected with a syringe 39, into an analyzer 40.

An example of a balloon catheter arrangement that can be configured and used as taught here is disclosed in Wang, U.S. 5,254,089. In that system, the sample space is provided as a series of lumens that are in the walls of the balloon itself. The lumens communicate with openings on the outer surface of the balloon. The lumens in the balloon wall are connected to a manifold that communicates with a lumen extending through the catheter to the proximal end. Another balloon catheter arrangement is described in WO-A-9211895 . In that device a balloon with apertures is positioned over another balloon with space therebetween.

In embodiments, the characteristics of the membrane can be selected based on the type of bodily sample to be taken. For example, the size of the openings can be selected to collect bodily fluid samples and reject cell samples. In this case, the openings may be about 0.5 to 0.1 micron, which permits fluid to enter but prevents cells from entering. On the other hand, the openings may be selected so that cells may enter. For example, the size of the openings may be about 50 to 75 µm. In some embodiments, in which the openings are too small to allow cells to pass, cell samples may nevertheless be taken in some cases because the cells can become attached to the surface by a wedging effect that occurs when cells are drawn partially into the pores.

The membrane need not cover the entire surface of the balloon. Rather, only a portion, e.g. one half (Fig. 1) or one third of the surface may be covered; the covered surface is then aligned with a corresponding target (e.g. diseased) portion of the vessel wall. The balloon can include several, spatially separated membranes with isolated, non-communicating storage spaces. The membranes can be used to take samples from different sites in the vessel in a sequential fashion without removing the catheter from the vessel.

In embodiments, a hydrogel polymer may be provided on the exterior of the membrane to increase lubricity. Suitable polymers are described in Sahatjian et al. U.S. 5,135,516, Fan U.S. 5,091,205 and Sahatjian, "Drug Delivery System", US-A-5,304,121. A swellable or sponge-like polymer can be provided in the storage space to hold the sample.

Taking samples by suction force is advantageous since it is a gentle removal that effectively sloughs material, e.g. cells, fluid from the surface and does not subject the body to the great trauma associated with severing or abraiding.

In another example, the balloon may also be moved slightly axially to lightly brush the membrane against the surface, which gently sloughs the sample from the surface. This latter embodiment is preferably carried out with a hydrogel coating over the membrane since its high lubricity, low frictional coefficient characteristics allow sample collection without excessive scraping of the surface. The sample may collect in the pore openings and may not enter the spaces in the body of the polymer. In embodiments, the sample is taken simultaneously with the dilatation of a stenosis. In this case, the membrane is provided on a dilation balloon and the balloon is inflated to dilatation pressures, e.g. 8-10 atmospheres. The invention is also applicable to areas other than the vascular system, such as the lungs or gastrointestinal tract, the urinary tract, the reproductive tract, or other parts of the body; especially those that can be accessed percutaneously by a catheter or like device.

The samples collected may be analyzed by techniques that can give a physician important information in determining or monitoring treatment. For example, a bodily sample of artherosclerotic plaque, endothelial cells, or chemical messengers, can be taken from an occluded region of an artery, particularly the coronary arteries or peripheral vessels, and analyzed to determine whether an injury has occured and, also the nature of the injury. For example, the analysis can determine whether the occlusion is highly calcified, or highly cholesteric, or highly fibrotic. These types of lesions may be differentiated based on cell samples or chemical indicators such as enzymes or proteins that are precursors to, for example, proliferation. DNA and RNA samples may as well be analyzed to the same effect. Analyses may be done by visual inspection, chemical analyses or spectral analyses, and by using methods such as gel permeation chromotography, infrared spectroscopy, electrophoresis, and micro analytical techniques. For example, plaque recognition by laser excited fluorescence spectroscopy is discussed by Bertorelli et al., *JACC*, Vol. 17, No. 6, May 1991, p. 160B. In some cases, the type of sample may be determined by visual observation by a physician. An example is a highly calcified sample. Samples, particularly cell samples, can also be analyzed for malignancy.

This information can determine what course of treatment should be followed. For example, if the sample is highly calcified, a laser ablation treatment may be the most effective in removing the occlusion. If highly cholesteric, a cholesterol dissolving drug may be delivered to the site or systemically. If highly fibrotic, an antiproliferative drug may be delivered. Another possible treatment is gene therapy including the delivery of antisense biochemical drugs. Gene therapy is discussed, for example, in Nabel et al., *JACC*, Vol. 17, No. 6, May 1991, 189B-94B. A drug delivery system is described in US-A-5,304,121. Drug delivery is also discussed in Wang U.S. 5,254,089.

In cases where cells are obtained using the methods and devices described above, in addition to or instead of analysis, the cells may be cultured outside the body and then placed back into the patient. For example, the cells may be placed back into the patient as an autologous coating on a graft or stent. The cells may also be placed back into the body using the device described above by disposing them on or through the membrane outside the body, delivering the device to position the membrane at a desired site inside the body, then releasing the cells from the membrane by applying flushing force as discussed, for example, with respect to drug delivery in Wang U.S. 5,254,089 supra. The cells may also be altered, e.g. genetically, before placing them back into the body.

Another technique for collecting samples is discussed in an application filed on the same day as this application by Sahatjian entitled "Bodily Sample Collection".

Still further embodiments are within the following claims. For example, methods and apparatus described above can be constructed and adapted for taking samples from parts of the body other than the vascular system.

## Claims

1. A sample collection device (2) for collecting a sample from a desired location in a patient, the sample collection device comprising a sampling probe in the form of an elongate catheter (4) having:
a proximal portion that remains outside the body and a distal portion that can be located at the desired location;
an expandable member (8) located at said distal portion, the expandable member being capable of being selectively expanded to larger diameters and contracted to smaller diameter;
a source of suction force (22) located at said proximal portion of the device; **characterized in that** it comprises
a membrane (16) located on at least a part of an outside surface of said expandable member;
a sample storage space (19) located in the interior of said membrane and communicating with source of suction force (22);
a plurality of support struts (17) in the space (19) to prevent the membrane (16) from collapsing during application of the suction force (22); and
a plurality of openings (18) extending through said membrane and communicating with the space in the interior of the membrane.

2. A device according to claim 1, further comprising an analysis apparatus (40) for facilitating analysis of said sample to determine physiological function.

3. A device according to claim 1, wherein the probe is in the form of an elongate vascular catheter (4).

4. A device according to claim 1, wherein the probe is in the form of an angioplasty catheter (4) and the expandable member is in the form if an inflatable balloon (8), the membrane (16) being disposed over said balloon.

## Patentansprüche

1. Probensammelvorrichtung (2) zum Sammeln einer Probe von einem gewünschten Ort in einem Patienten, wobei die Probensammelvorrichtung eine Probensonde in der Form eines länglichen Katheters (4) umfasst, welcher aufweist:
einen nahen Abschnitt, der außerhalb des Körpers verbleibt und einen entfernten Abschnitt, der am gewünschten Ort lokalisiert werden kann;
ein expandierbares Bauteil (8) lokalisiert an dem entfernten Abschnitt, wobei das expandierbare Bauteil dazu in der Lage ist selektiv auf größere Durchmesser expandiert zu werden und auf kleinere Durchmesser kontrahiert zu werden;
eine Quelle von Saugkraft (22) lokalisiert an dem nahen Abschnitt der Vorrichtung;
**dadurch gekennzeichnet, dass** sie aufweist,
eine Membran (16) lokalisiert an wenigstens einem Teil einer Außenoberfläche des expandierbaren Bauteils;
einen Probenspeicherraum (19) lokalisiert im Inneren der Membran und in Verbindung stehend mit der Quelle von Saugkraft (22);
eine Vielzahl von Stützstreben (17) in dem Raum (19), um zu verhindern, dass die Membran (16) während der Anwendung von Saugkraft (22) kollabiert; und
eine Vielzahl von Öffnungen (18), die sich durch die Membran erstrecken und in Verbindung stehen mit dem Raum im Inneren der Membran.

2. Vorrichtung gemäß Anspruch 1, weiter aufweisend eine Analysevorrichtung (40) zur Erleichterung der Analyse der Probe, um physiologische Funktionen zu bestimmen.

3. Vorrichtung gemäß Anspruch 1, wobei die Sonde die Gestalt eines länglichen vaskulären Katheters (4) aufweist.

4. Vorrichtung gemäß Anspruch 1, wobei die Sonde die Gestalt eines Angioplastiekatheters (4) aufweist, und das expandierbare Bauteil die Form eines aufblasbaren Ballons (8) aufweist, wobei die Membran (16) über diesem Ballon angeordnet ist.

## Revendications

1. Dispositif de collecte d'échantillons (2) destiné à collecter un échantillon dans un endroit souhaité chez un patient, le dispositif de collecte d'échantillons comprenant une sonde d'échantillonnage sous la forme d'un cathéter allongé (4) ayant :
une portion proximale qui reste à l'extérieur du corps et une portion distale qui peut être située à l'emplacement souhaité ;
un élément étirable (8) situé au niveau de ladite portion distale, l'élément étirable étant capable d'être étiré de manière sélective à des diamètres plus grands et contracté à un diamètre plus petit ;
une source de force d'aspiration (22) située au niveau de ladite portion proximale du dispositif ; **caractérisé en ce qu'**il comprend
une membrane (16) située sur au moins une partie d'une surface extérieure dudit élément étirable ;
un espace de stockage des échantillons (19) situé à l'intérieur de ladite membrane et communiquant avec la source de force d'aspiration (22) ;
une pluralité de plaquettes de support (17) dans l'espace (19) pour éviter que le membrane (16) tombe pendant l'application de la force d'aspiration (22) ; et
une pluralité d'ouvertures (18) s'étendant à travers ladite membrane et communiquant avec l'espace à l'intérieur de la membrane.

2. Dispositif selon la revendication 1, comprenant en outre un appareil d'analyse (40) pour faciliter l'analyse dudit échantillon pour déterminer la fonction physiologique.

3. Dispositif selon la revendication 1, dans lequel la sonde est sous la forme d'un cathéter vasculaire allongé (4).

4. Dispositif selon la revendication 1, dans lequel la sonde est sous la forme d'un cathéter d'angioplastie (4) et l'élément étirable est sous la forme d'un ballon gonflable (8), la membrane (16) étant disposée sur ledit ballon.
